(19) Europäisches Patentamt / European Patent Office / Office européen des brevets

(11) **EP 4 425 428 B1**

(12) **EUROPEAN PATENT SPECIFICATION**

(45) Date of publication and mention
of the grant of the patent:
**03.12.2025 Bulletin 2025/49**

(51) International Patent Classification (IPC):
*G06T 7/246* (2017.01)

(21) Application number: **23187559.2**

(22) Date of filing: **25.07.2023**

(52) Cooperative Patent Classification (CPC):
**G06T 7/248;** G06T 2207/10121; G06T 2207/20081;
G06T 2207/20084; G06T 2207/30021;
G06T 2207/30101

(54) **TARGET TRACKING IN MEDICAL IMAGE DATA**

ZIELVERFOLGUNG IN MEDIZINISCHEN BILDDATEN

SUIVI DE CIBLE DANS DES DONNÉES D'IMAGE MÉDICALE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **02.03.2023 US 202363487961 P**

(43) Date of publication of application:
**04.09.2024 Bulletin 2024/36**

(73) Proprietor: **Siemens Healthineers AG
91301 Forchheim (DE)**

(72) Inventors:
• **ZHANG, Yue**
**Jersey City, 07310 (US)**
• **DEMOUSTIER, Marc**
**Princeton, 08540 (US)**
• **NARASIMHA MURTHY, Venkatesh**
**Hillsborough, 08844 (US)**
• **GHESU, Florin-Cristian**
**91054 Erlangen (DE)**
• **COMANICIU, Dorin**
**Princeton, 08540 (US)**

(74) Representative: **Kraus & Lederer PartGmbB
Thomas-Wimmer-Ring 15
80539 München (DE)**

(56) References cited:
• ZHANG GUIFANG ET AL: "A Temporary Transformer Network for Guide- Wire Segmentation", 2021 14TH INTERNATIONAL CONGRESS ON IMAGE AND SIGNAL PROCESSING, BIOMEDICAL ENGINEERING AND INFORMATICS (CISP-BMEI), IEEE, 23 October 2021 (2021-10-23), pages 1 - 5, XP034039569, DOI: 10.1109/CISP-BMEI53629.2021.9624350
• YAN BIN ET AL: "Learning Spatio-Temporal Transformer for Visual Tracking", 2021 IEEE/CVF INTERNATIONAL CONFERENCE ON COMPUTER VISION (ICCV), IEEE, 10 October 2021 (2021-10-10), pages 10428 - 10437, XP034093656, DOI: 10.1109/ICCV48922.2021.01028
• CHEN BOR-JENG ET AL: "Guidewire tracking using a novel sequential segment optimization method in interventional X-ray videos", 2016 IEEE 13TH INTERNATIONAL SYMPOSIUM ON BIOMEDICAL IMAGING (ISBI), 1 April 2016 (2016-04-01), pages 103 - 106, XP093179572, DOI: 10.1109/ISBI.2016.7493221
• HUA MA ET AL: "Dynamic Coronary Roadmapping via Catheter Tip Tracking in X-ray Fluoroscopy with Deep Learning Based Bayesian Filtering", ARXIV.ORG, CORNELL UNIVERSITY LIBRARY, 201 OLIN LIBRARY CORNELL UNIVERSITY ITHACA, NY 14853, 11 January 2020 (2020-01-11), XP081577386, DOI: 10.1016/J.MEDIA.2020.101634

**Description**

TECHNICAL FIELD

Various examples of the disclosure generally pertain to tracking a target in medical imaging data. For example, a catheter tip can be tracked. Various examples specifically pertain to a processing pipeline employing machine-learning algorithms for tracking the target in the medical imaging data.

BACKGROUND

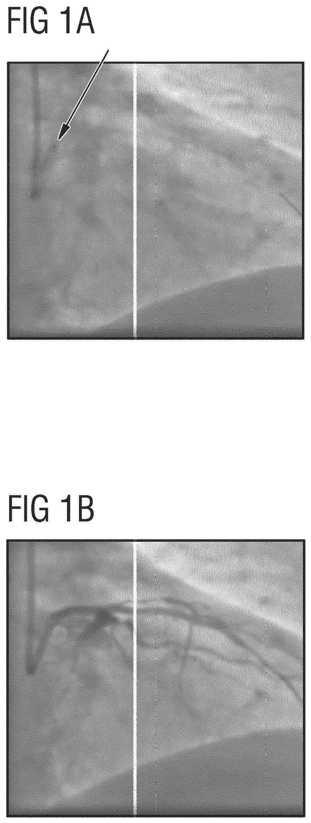

Tracking of interventional devices plays an important role in aiding surgeons during catheterized interventions such as percutaneous coronary interventions (PCI), cardiac electrophysiology (EP), or trans arterial chemoembolization (TACE). In cardiac image-guided interventions, surgeons can benefit from visual guidance provided by mapping vessel information from fluoroscopy (cf. FIG. 1A where a catheter tip is shown; marked by the arrow) to angiography (cf. FIG. 1B; angiographic images are more challenging, at times the catheter tip is occluded by the contrast agent.). See Ref. [7] and Ref. [11].

The catheter tip is used as an anchor point representing the root of the vessel tree structure. This visual feedback helps in reducing the contrast usage for visualizing the vascular structures and it can also aid in effective placements of stents or balloons. See Ref. [8].

Tracking catheter tip can also provide significant value to co-register intravascular ultrasound (IVUS) and angiography to enable the detailed analysis of vessel, lumen and wallstructure. See Ref. [1] and Ref. [12].

Recently, deep learning-based Siamese networks have been proposed for medical device tracking, see Refs. [2,5,6]. These networks achieve high frame rate tracking but are limited by their online adaptability to changes in target's appearance as they only use spatial information. Cycle Ynet - see Ref. [6] - uses the cycle consistency of a sequence and adds a semi-supervised learning approach by doing a forward and a backward tracking, but this suffers from drifting for long sequences and cannot recover from misdetections because of single template usage. Ref. [7] uses Convolutional Neural Network (CNN) followed by particle filtering as a post processing step. The drawback of this method is that it doesn't compensate for the cardiac and respiratory motions as there is no explicit motion model for capturing temporal information. A similar method adds a graph convolutional neural network for aggregating both spatial information and appearance features - see Ref. [4] - to provide a more accurate tracking but its effectiveness is limited by its vulnerability to appearance changes and occlusion resulting from detection techniques. Optical flow-based network architectures - see Ref. [9] - utilize keypoint tracking throughout the entire sequence to estimate the motion of the whole image. However, such approaches are not adapted for tracking a single point, such as a catheter tip.

For general computer vision applications, Transformer based-trackers - see Ref. [10] - have achieved state-of-the-art performance, see Ref. [3,13,14]. Initially proposed for Natural Language Processing (NLP), transformers learn the dependencies between elements in a sequence, making it intrinsically well suited at capturing global information.

STARK - see Ref. [22] - is among the top performers but one of the key issues of these networks is that they are trained on extensively annotated datasets of natural images, which make their application to medical data challenging, as the annotations are less abundant. Regarding catheter tip tracking or more generally device tracking on X-ray images some methods have been developed. For example, Cycle Tracker solves many issues as it does not require extensively annotated datasets to perform robust tracking. The main idea of this network is to use weakly supervised tracking-by-matching by decomposing the tracking in two steps: forward tracking and backward tracking. If one considers a video sequence of N frames, the target will first be tracked from frame 0 to N, then it will be tracked from frame N to 0. So, if the object is tracked correctly, then the backward tracking should bring the model at the starting position.

Ref. [28] suggests a method for guide-wire segmentation in X-ray fluoroscopy sequences using a temporary transformer network, taking the current frame and the previous frame as input.

SUMMARY

Accordingly, a need exists for advanced techniques of tracking a target. In particular, a need exists for advanced techniques of tracking a target in medical imaging data. A need exists for advanced tracking techniques that alleviate or mitigate at least some of the above-identified restrictions or drawbacks.

This need is met by the features of the independent claims. The dependent claims define embodiments.

To overcome the limitations of the prior art outlined above, a generic, end-to-end model / processing pipeline for target tracking is disclosed. The tracking can be dependent on at least one of a temporal context or a spatial context.

Multiple template images (containing the target) and a search image (where the target location is identified, usually the current frame) are input to the system.

Where multiple template images are used, they can have different perspective onto the target. Where multiple template images are used, they can have different occlusion degrees, e.g., with respect to a contrast agent that is administered.

Each of the one or more template images can have a smaller size and/or lower resolution than the search image. The one or template images can depict fewer context of the target if compared to the search image.

The system first passes them through a feature encoding network to encode them into the same feature space. Next, the features of template and search are fused together by a fusion network, e.g., a vision transformer network. The fusion network / model builds complete associations between the template feature and search feature and identifies the features of the highest association. The fused features are then used for target (e.g., catheter tip) and context prediction (e.g., catheter body). I.e., based on the fused features, a position prediction of the target is performed in the search image.

While such processing pipeline can learn to perform these two tasks together, spatial context information is offered implicitly to provide guidance to the target detection.

In addition to the spatial context, the proposed framework optionally also leverages the temporal context information which is generated, e.g., using a motion flow network. This temporal information helps in further refining the target location.

The processing pipeline can include a transformer encoder (vision transformer network) that helps in capturing the underlying relationship between template and search image using self and cross attentions, followed by multiple transformer decoders to accurately track catheter tip or another target.

A computer-implemented method of tracking a target in medical imaging data is disclosed. The method includes determining an encoded representation of a search image of the medical imaging data in a feature space. The encoded representation of the search image is determined using a feature encoding network. The search image depicts a target, as well as a surrounding of the target. The method also includes determining encoded representation of one or more template images of the medical imaging data in the feature space using the feature encoding network. The one or template images depict the target. The method also includes determining fused features. This is done by fusing the encoded representations of the one or template images and the encoded representation of the search image using a fusion network. The fusion network may build complete associations between the encoded representations and may identify features of highest association. The method further includes, based on the fused features, determining a position prediction of the target in the search image.

The medical imaging data can be determined using one or more of the following imaging modalities: fluoroscopy; angiography; X-ray.

The method further includes determining a segmentation of a context of the target in the search image based on the fused features. Then, the method further includes refining the position prediction of the target based on the segmentation of the context of the target device. I.e., a spatial context can be determined based on the fused features and considered in the tracking, e.g., by refining the position prediction.

A processing device includes a processor and a memory. The memory stores program code. The processor is configured to load and execute the program code. The processor, upon executing the program code is configured to perform a method of tracking a target in medical imaging data as disclosed above.

A program code is executable by a processor. The processor, upon executing the program code, is configured to perform a method of tracking a target in medical imaging data as disclosed above.

It is to be understood that the features mentioned above and those yet to be explained below may be used not only in the respective combinations indicated, but also in other combinations or in isolation without departing from the scope of the invention.

BRIEF DESCRIPTION OF THE DRAWINGS

FIG. 1A schematically illustrates a catheter tip location in a fluoroscopy medical imaging data.

FIG. 1B schematically illustrates a catheter tip location in an angiography medical imaging data.

FIG. 2 schematically illustrates a processing pipeline for target tracking according to various examples.

FIG. 3 schematically illustrates details with respect to a decoder network according to various examples.

FIG. 4 schematically illustrates a processing device according to various examples.

FIG. 5 is a flowchart of a method according to various examples.

## DETAILED DESCRIPTION OF EMBODIMENTS

Some examples of the present disclosure generally provide for a plurality of circuits or other electrical devices. All references to the circuits and other electrical devices and the functionality provided by each are not intended to be limited to encompassing only what is illustrated and described herein. While particular labels may be assigned to the various circuits or other electrical devices disclosed, such labels are not intended to limit the scope of operation for the circuits and the other electrical devices. Such circuits and other electrical devices may be combined with each other and/or separated in any manner based on the particular type of electrical implementation that is desired. It is recognized that any circuit or other electrical device disclosed herein may include any number of microcontrollers, a graphics processor unit (GPU), integrated circuits, memory devices (e.g., FLASH, random access memory (RAM), read only memory (ROM), electrically programmable read only memory (EPROM), electrically erasable programmable read only memory (EEPROM), or other suitable variants thereof), and software which co-act with one another to perform operation(s) disclosed herein. In addition, any one or more of the electrical devices may be configured to execute a program code that is embodied in a non-transitory computer readable medium programmed to perform any number of the functions as disclosed.

In the following, embodiments of the invention will be described in detail with reference to the accompanying drawings. It is to be understood that the following description of embodiments is not to be taken in a limiting sense. The scope of the invention is not intended to be limited by the embodiments described hereinafter or by the drawings, which are taken to be illustrative only.

The drawings are to be regarded as being schematic representations and elements illustrated in the drawings are not necessarily shown to scale. Rather, the various elements are represented such that their function and general purpose become apparent to a person skilled in the art. Any connection or coupling between functional blocks, devices, components, or other physical or functional units shown in the drawings or described herein may also be implemented by an indirect connection or coupling. A coupling between components may also be established over a wireless connection. Functional blocks may be implemented in hardware, firmware, software, or a combination thereof.

Hereinafter, techniques for tracking a target are disclosed. Various kinds and types of targets can be tracked. Medical device targets can be tracked in medical imaging data. One particular use case is percutaneous coronary intervention (PCI). PCI is a non-surgical procedure that uses a catheter (a thin flexible tube) to place a small structure called a stent in order to restore normal blood flow circulation in the event of obstruction of a coronary artery. During this minimally invasive procedure, the catheter is guided under radioscopic control to the affected coronary artery using two types of X-ray, fluoroscopy, and angiography. Since the arteries are not visible on fluoroscopic images, contrast medium is injected several times to facilitate the intervention, the PCI procedure is related to potentially high levels of radiation exposure and, therefore, greater risk of radiation-induced side effects. See Ref. [21].

But still, to limit radiation exposure, interventional cardiologists have to rely on X-ray fluoroscopic images in which the coronary artery are no longer contrast-filled, which involves mentally recreating the coronary arterial tree. Moreover, when using a contrast medium, the catheter is completely obstructed, which complicates the intervention and can increase intervention time, thus increase the amount of radiation exposure. Assistance can be provided by tracking the catheter tip onto live X-ray fluoroscopic and angiographic images.

Techniques for device tracking in X-ray fluoroscopy (cf. FIG. 1A) and angiography (cf. FIG 1B) using spatio-temporal context guidance are described in detail. It can provide visual navigation assistance and be used for Dynamic Coronary Roadmapping (DRM) to infer the compensation of breathing and heartbeat motions, which has the potential to reduce procedure time, and by consequence, reduce the radiation exposure.

While catheter-tip tracking is disclosed in detail, other types of devices can also be tracked using the techniques disclosed herein. For instance, certain anatomical structures can be tracked. Other interventional medical instruments can be tracked.

The present disclosure provides for a generic model framework (processing pipeline) for target tracking. A template image (containing the target) and a search image (where the target location is identified, usually the current frame) are input to the processing pipeline. The pipeline first passes them through a feature encoding network to encode them into the same feature space. Next, the features of template and search are fused together by a fusion network, e.g., vision transformer. The fusion model builds complete associations between the template feature and search feature and identify the features of highest association. The fused features are then used for target and context prediction. As a general rule, for medical images in the PCI use case, the context can be the device that the target attaches onto (e.g., catheter tip and body), neighboring anatomical structures, and/or neighboring devices in field of view. A detection-segmentation module is used for target detection and context segmentation. While this module learns to perform these two tasks together, spatial information from context is offered implicitly to provide guidance to the target detection. See Ref. [27]. The proposed processing pipeline then preferably leverages the temporal information of the context (i.e., context flow), generated through a motion flow network, and uses this information to refine the target location. The context flow, together with any spatial prior knowledge of the context, is used to refine the context segmentation through a context refinement module. The refined context segmentation is used in target tracking in next frames.

Using temporal context information is based on the finding that, in interventional procedures, one common challenge for visual tracking comes from occlusion. This can be caused by injected contrast medium (in the angiographic image) or interferring devices such as sternal wires, stent and additional guiding catheters. If target is occluded in the search image, using only spatial information for localization is inadequate. To address this challenge, according to the disclosed techniques, a motion prior of the target to is used to further refine the tracked location. As the target is a sparse object, this can be preferably done via optical flow estimation of the context.

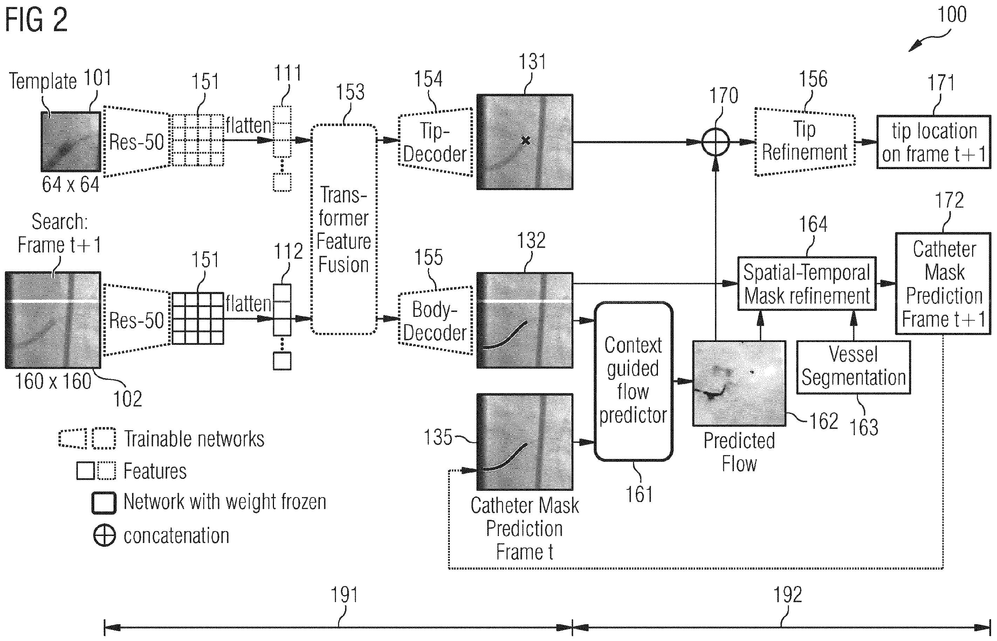

FIG. 2 schematically illustrates a processing pipeline 100 according to various examples. The processing pipeline 100 includes a first stage 191 and a second stage 192. The first stage 191 is for localization of the target. A vision transformer network can be used. The second stage 192 is for refinement of this localization.

A template image 101 and search image 102 are first being encoded into the same feature space with feature encoder network 151, e.g., Res-50 network. The features (encoded representations 111, 112 of the images 101, 102) are then forwarded through a vision transformer network 153 for feature fusion. The vision transformer network 153 builds a complete association of the template and search features with multi-head attention module that is built in. The fused features are then forwarded into a tip decoder network 154 and a body decoder network 155 for initial tip localization and catheter body segmentation, respectively.

Taking the segmented catheter body 135 from previous frame, a flow prediction module 161 is employed to predict the optical flow 162 of the context, i.e., the motion of the segmentation mask of the catheter body.

The flow prediction module 161 can be implemented using prior-art techniques, e.g., Lucas-Kanade method or Horn-Schunck method. It would also be possible to use a neural network to calculate the optical flow.

This predicted motion indicated by the optical flow map 162 indicates the apparent motion of the catheter from the perspective of the imaging device. By the geometrical relation between the catheter body and tip, such motion information of catheter body aids the prediction of the motion for the catheter tip.

In the processing pipeline 100, the predicted optical flow map 162 is concatenated (at node 170) together with the initial catheter tip localization map 131 (output of the tip-decoder network 154; can also be referred to as catheter tip localization mask 131) and forwarded through a tip refinement network 156 to finalize the tip location on current search frame. The finalized position prediction is output as map 171.

For the catheter body segmentation, the predicted catheter body segmentation map 132, the optical flow map 162 and, optionally, a predicted vessel segmentation map 163 are input to a Spatial-Temporal Mask refinement block 164.

The vessel segmentation map 163 can be determined using prior-art techniques, e.g., a pretrained model, and helps to remove the potential vessels segmented along the catheter body due to the contrast medium.

This refinement block 164 helps to get a cleaner segmentation mask for angiographic images especially. This refinement block 164 combines both spatial information (segmentation map 132) and temporal information (optical flow map 162) to keep a clean segmentation of the catheter through a long sequence.

This segmentation mask 172 of the context output from the refinement block 164 will be used in prediction of target in next frame (dotted feedback line).

Next, an example implementation of the feature fusion using the feature encoding network 151 in the first stage 191 for localization of the target are described.

In the encoding stage, given a set of template image patches centered around the target $\{T_{ti}\}_{ti \in H}$ and current frame $I_s$ as the search image. The target location is determined by fusing information from the multiple templates. This can be naturally accomplished by mutli-head attention. Specifically, the ResNet encoder is denoted by $\theta$, given the feature map of the search image $\theta(f_s)$ $\mathbb{R}^{C \times H_s \times W_s}$, and the feature maps of the templates $\{\theta(T_{ti})\}$, we use 1x1 convolutions to project and flatten them into d-dimensional vector query key and value embedding, $q_s, k_s, v_s$ for the search image features and $\{q_{ti}\}, \{k_{ti}\}, \{v_{ti}\}$ for templates features respectively. The attention is based on the concatenated vectors,

$$\text{Attention}(Q, K, V) := \text{softmax}(\frac{QK^T}{\sqrt{d}})V$$

where $Q = \text{Concat}(q_s, q_{t1}, q_{t2}, ... q_{tn})$, $K = \text{Concat}(k_s, k_{t1}, k_{t2}, ... k_{tn})$, $V = \text{Concat}(v_s, v_{t1}, v_{t2}, ..., v_{tn})$. The definition of the multi-head attention then follows Ref. [10].

Next, details with respect to the decoder networks 154, 155 in the first stage 191 for localization of the target are explained.

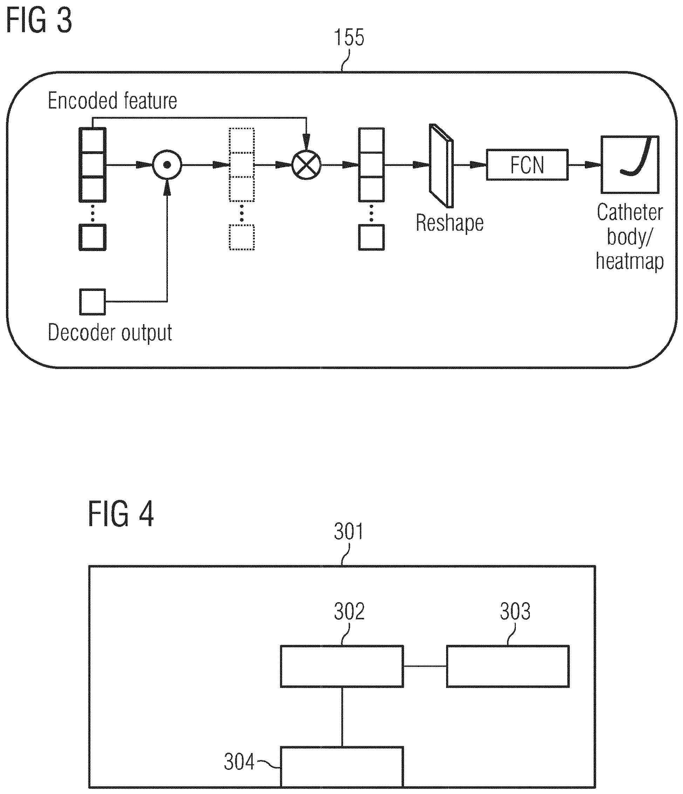

In the decoding stage, the transformer decoder is adjusted to multi-task setting. As the catheter tip represents a sparse object in the image, solely detecting itself suffers from class imbalance issue. To guide catheter tip tracking with spatial information, additional contextual information is incorporated by simultaneously segmenting the catheter body in the same frame. Specifically, two object queries $(e_1, e_2)$ are employed in the decoder where $e_1$ defines the position of the catheter tip, and $e_2$ defines the mask of the catheter body. As illustrated in Fig. 3, similarity scores are first calculated between the decoder and the encoder output via dot product. Then, element-wise product between the similarity scores and the encoder features is used to promote regions with high similarity. After reshaping the processed features to $d \times H_s \times W_s$, an encoder-decoder structured 6-layer FCN is attached to process the features to probability maps with the same size as the search image. A combination of the binary cross-entropy and the dice loss is then used,

$$L = \lambda_{bce}^x L_{bce}(G(x_i; \mu, \sigma), \hat{x}_i^s) + \lambda_{dice}^x L_{dice}(G(x_i; \mu, \sigma), \hat{x}_i^s) + \lambda_{bce}^m L_{bce}(m_i, \hat{m}_i) + \lambda_{dice}^m L_{dice}(m_i, \hat{m}_i)$$

Where $x_i$, $m_i$ represents the ground truth annotation of the catheter tip and mask, $\hat{x}_i^s, \widehat{m}_i^s$ are predictions respectively. Here we use sup-script "s" to denote the predictions from this spatial stage. $G(x_i; \mu, \sigma) := \exp(-\| x_i - \mu \|^2 / \sigma^2)$ is the smoothing function that transfers dot location of $x_i$ to probability map. $\lambda_{bce}^*$ , $\lambda_{dice}^* \in \mathbb{R}$ are hyperparameters.

Next, details of the second stage 192 for refinement of the localization are explained. The employed techniques are based on the finding that obtaining ground truth optical flow in real world data is a challenging task and may require additional hardware such as a motion sensor. Training a model for optical flow estimation directly in the image space is then difficult. Different to such reference implementations, the processing pipeline 100 estimates the flow in the segmentation space, i.e., on the predicted heatmaps of the catheter body between neighboring frames. This is based on the RAFT model, see Ref. [9].

Specifically, given the predicted segmentation maps $m_{t-1}$ and $m_t$ a 6-block ResNet encoder $g_\theta$ is used to extract the features $g_\theta(m_{t-1})$, $g_\theta(m_t) \in R^{Hf \times Wf \times Df}$. Then, the correlation volume pyramid $\{C_i\}_{i=0}^3$ is constructed, where

$$C_i = \text{AvgPool}(corr(g_\theta(m_{t-1}), g_\theta(m_t)), \text{stride} = 2^i).$$

Here $corr(g_\theta(m_{t-1})\ g_\theta(m_t) \in \mathbb{R}^{H_f \times W_f \times H_f \times W_f}$ stands for correlation evaluation

$$corr(g_\theta(m_{t-1}), g_\theta(m_t)_{ijkl} = \sum_{h=1}^{D_f} g_\theta(m_{t-1})_{ijh} \cdot g_\theta(m_t)_{klh},$$

which can be computed via matrix multiplication. Starting with an initial flow $f_0 = 0$, the same model setup as Ref. [9] is followed to recurrently refine the flow estimates to $f_k = f_{k-1} + \Delta f$ with a gated recurrent unit (GRU) and a delta flow prediction head of two convolutional layers. Given the tracked tip result from previous frame $\hat{x}_{t-1}$, it is possible to then predict the new tip location at time $t$ by warping with context flow $\hat{x}_t^f = f^k \circ \hat{x}_{t-1}$. Here, we use sup-script "$f$" to denote the prediction by flow warpping.

Since the segmentations of the catheter body are sparse objects compared to the entire image, computation of the correlation volume and subsequent updates can be restricted a cropped-sub-image which reduces computation cost and flow interference time. As the flow estimation is performed on segmentation map, one can simply generate synthetic flows and warp them with the existing catheter body annotation to generate data for model training.

Next, details with respect to the refinement block 164 are explained.

Score map with combined information from the spatial localization stage and the temporal prediction by context flow is generated:

$$S_t(u, v) = \begin{cases} (\alpha + \hat{m}_t^s(u, v))(\hat{x}_t^s(u, v) + \hat{x}_t^f(u, v)) & \hat{m}_t^s(u, v) > 0, \\ \hat{x}_t^s(u, v) + \hat{x}_t^f(u, v) & \text{otherwise.} \end{cases}$$

Here $\alpha$ is a positive scalar. It helps the score map to promote coordinates that are activated at all three maps, the spatial prediction $\hat{x}_t^s$, temporal prediction $\hat{x}_t^f$ and the context $\widehat{m}_t^s$. Finally, the score map is forwarded through the refinement block 164 to finalize the prediction. The refinement block 164 or module consists of a stack of three convolutional layers. Similar to the spatial localization stage, a combination of the binary cross-entropy and the dice loss is used as the final loss.

Summarizing, using the processing pipeline 100, based on a sequence of consecutive X-ray images $\{I_t\}_{t=1}^n$

(the search images 102) and an initial location of the target catheter tip $x_0=(u_0, v_0)$, (identified in the template image 101) the location of the target $x_t=(u_t, v_t)$, is tracked at any time $t, t > 1$. The proposed model framework includes two stages, target localization stage 191 and motion refinement stage 192. First, given a selective set of template image patches 101 and the search image 102, their high-level features with share-weighted residual network encoding are obtained and it is possible to leverage a transformer encoder 153 to build complete feature point association. Followed by a modified transformer decoder 154 and 155 to jointly localize the target and segment the neighboring context, i.e., body of the catheter. Next, the context motion is estimated via optical flow on the catheter body segmentation between neighboring frames and use this to refine the detected target location. Finally, confident predictions are added into the set of templates and use together the context segmentation for target tracking in the next frame.

FIG. 4 schematically illustrates a processing device 301 according to various examples. The processing device 301 includes a processor 302, e.g., a CPU or GPU. The processing device 301 also includes a memory 303 that stores program code that is executable by the processor 302. The processing device 301 also includes a communication interface 304 via which the processor 302 can retrieve medical imaging data, e.g., 2D imaging data or 3D imaging data acquired various imaging modalities, e.g., x-ray. The processor 302 is configured to perform techniques as disclosed herein when executing the program code stored in the memory 303.

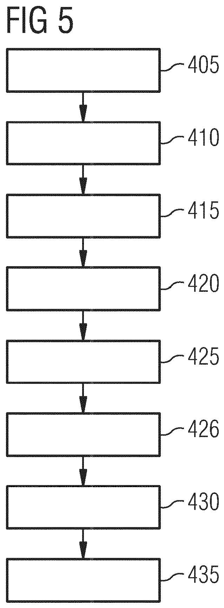

FIG. 5 is a flowchart of a method according to various examples. For example, the method of FIG. 5 can be executed by a processing device such as the processing device 301 of FIG. 4. For instance, the method of FIG. 5 can be executed by the processor 302 based on program code that is stored in the memory 303.

The method of FIG. 5 enables tracking of a target, e.g., a catheter tip. A vision transformer network can be used, thereby increasing accuracy and robustness of the tracking. A spatial context information can be used, e.g., for refining a prediction of the target location. For instance, a context mask and vessel segmentation can be considered as spatial context information. I.e., a neighboring context of the target is taken into account to refine the localization of the target. A temporal context information can be optionally taken into account, e.g., an optical flow based on a segmentation mask. Taking into account spatial context information and/or temporal context information has advantages over prior art solutions - e.g., Ref. [6] - where only information of the target itself is considered. Thus the proposed solution is more robust in cases with target occlusion and distraction (e.g. tip from a close but different catheter).

In box 405, an encoded representation of a search image of medical imaging data is determined in a feature space. This is done using an encoding network. The search image depicts a target, e.g., a catheter tip, and a surrounding of the target. Details with respect to such encoding network have been explained in connection with FIG. 2: encoding network 151 and the search image 102.

Next, in box 410, encoded representations of one or more template images of the medical imaging data are determined in the feature space, using the same feature encoding network. Respective details have also been explained in connection with FIG. 2.

Then, at box 415, fused features are determined by fusing the encoded representation of the one or more template images and the encoded representation of the search image using a fusion network, cf. FIG. 2: fusion network 153. The fusion network can be implemented by a vision transformer network.

At box 420, a position prediction of the target in the search image is determined based on the fused features. Details with respect to such localization have been disclosed in connection with the decoder network 154 in connection with FIG. 2. The position prediction is a first estimate that can be refined in downstream processing of the processing pipeline.

At box 425, it is possible to determine a segmentation of a context of the target in the search image, cf. decoder network 155 in FIG. 2. The segmentation map 132 depicts the catheter body. The segmentation of the context of the target can then be used to refine the position prediction of the target, box 430.

In detail: While above in connection with FIG. 2 scenarios have been disclosed in which the spatial context is used to determine the optical flow map, i.e., to determine a spatiotemporal context, in other variants, it would also be possible that the spatial context is used without a temporal context. The temporal context is, hence, optional. Without the temporal context, it would be possible to use a refinement network that obtains, as an input, both the segmentation map 131 indicating the localization of the target, as well as the segmentation map 132 indicating the context (e.g., by concatenating the map 131 and the map 132 and inputting to a respective refinement network). Then, spatial consistency between the localization of the target, as well as the localization of the context of the target can be enforced. However, in some examples, at box 426, an optical flow map is determined based on the segmentation of the context and one or previous segmentations of the context and then the position prediction of the target is refined based on the optical flow map at box 430. This can be based on a refinement network 156 as illustrated in FIG. 2. The refinement network 156 operates based on the position prediction of the target, catheter tip localization map 131) and the optical flow map 162, e.g., using the concatenation at 170.

At optional box 435, it is possible to refine the segmentation of the context of the target based on the optical flow. Respective techniques have been discussed in connection with FIG. 2: spatial-temporal refinement block 164. This is helpful where the context segmentation is then used in the subsequent iteration to determine the optical flow. The refinement of the segmentation of the context can take into account additional information such as the vessel segmentation map 163, cf. FIG. 2.

Although the invention has been shown and described with respect to certain preferred embodiments, equivalents and modifications will occur to others skilled in the art upon the reading and understanding of the specification. The present invention includes all such equivalents and modifications and is limited only by the scope of the appended claims.

For illustration, while various examples have been disclosed in the context of catheter tip tracking, the proposed processing pipeline can be applied in general device tracking in both cardiac and neuro interventional image-guided therapies. The catheter body segmentation and flow prediction can be replaced with any device or structure that has a direct impact on the motion of the target. One can use the proposed approach to retrieve the motion of such structures, and learn a refinement module to refine the target location based on the neighboring structure motion. Various interventional devices / medical instruments can be tracked; the context can be the body of the interventional medical instrument extending away from the tip. However, also other types of context can be considered, e.g., anatomical features in the surrounding of the target.

For further illustration, various examples have been disclosed in the framework of fluoroscopy and angiography medical image data. However, the disclosed techniques can be employed to variety of image modalities, include but not limited to X-ray and ultrasound. In 3D images such as TEE, TTE and ICE, all convolutional networks will be implemented with 3D convolutions. The predicted context flow then indicates 3d motions of the neighboring structures of the target.

REFERENCES

1. Araki, T., Ikeda, N., Dey, N., Chakraborty, S., Saba, L., Kumar, D., Godia, E.C., Jiang, X., Gupta, A., Radeva, P., Laird, J.R., Nicolaides, A., Suri, J.S.: A comparative approach of four different image registration techniques for quantitative assessment of coronary artery calcium lesions using intravascular ultrasound. vol. 118,pp. 158-172 (2015)

2. Bromley, J., Guyon, I., LeCun, Y., Säckinger, E., Shah, R.: Signature verification using a "siamese" time delay neural network. In: Cowan, J., Tesauro, G., Alspector, J. (eds.) Advances in Neural Information Processing Systems. vol. 6. MorganKaufmann (1993)

3. Chen, Q., Wu, Q., Wang, J., Hu, Q., Hu, T., Ding, E., Cheng, J., Wang, J.: Mixformer: Mixing features across windows and dimensions. In: Proceedings of the IEEE/CVF Conference on Computer Vision and Pattern Recognition (CVPR). pp. 5249-5259 (2022)

4. Huang, L., Liu, Y., Chen, L., Chen, E.Z., Chen, X., Sun, S.: Robust landmarkbased stent tracking in x-ray fluoroscopy. In: Avidan, S., Brostow, G., Ciss'e, M., Farinella, G.M., Hassner, T. (eds.) Computer Vision - ECCV 2022. pp. 201-216. Springer Nature Switzerland, Cham (2022)

5. Li, B., Yan, J., Wu, W., Zhu, Z., Hu, X.: High performance visual tracking with siamese region proposal network. In: 2018 IEEE/CVF Conference on Computer Vision and Pattern Recognition. pp. 8971-8980 (2018)

6. Lin, J., Zhang, Y., Amadou, A.a., Voigt, I., Mansi, T., Liao, R.: Cycle ynet: Semisupervised tracking of 3d anatomical landmarks. In: Liu, M., Yan, P., Lian, C., Cao, X. (eds.) Machine Learning in Medical Imaging. pp. 593-602. Springer International Publishing, Cham (2020)

7. Ma, H., Smal, I., Daemen, J., van Walsum, T.: Dynamic coronary roadmapping via catheter tip tracking in x-ray fluoroscopy with deep learning based bayesian filtering. vol. 61, p. 101634 (2020)

8. Piayda, K., Kleinebrecht, L., Afzal, S., Bullens, R., ter Horst, I., Polzin, A., Veulemans, V., Dannenberg, L., Wimmer, A.C., Jung, C., Bönner, F., Kelm, M., Hellhammer, K., Zeus, T.: Dynamic coronary roadmapping during percutaneous coronary intervention: a feasibility study. vol. 23, p. 36 (2018) 9. Teed, Z., Deng, J.: Raft: Recurrent all-pairs field transforms for optical flow. In: Vedaldi, A., Bischof, H., Brox, T., Frahm, J.M. (eds.) Computer Vision - ECCV 2020. pp. 402-419. Springer International Publishing, Cham (2020)

10. Vaswani, A., Shazeer, N., Parmar, N., Uszkoreit, J., Jones, L., Gomez, A.N., Kaiser, L.u., Polosukhin, I.: Attention is all you need. In: Guyon, I., Luxburg, U.V., Bengio, S., Wallach, H., Fergus, R., Vishwanathan, S., Garnett, R. (eds.) Advances in Neural Information Processing Systems. vol. 30. Curran Associates, Inc. (2017)

11. Wang, P., Chen, T., Ecabert, O., Prummer, S., Ostermeier, M., Comaniciu, D.: Image-based device tracking for the co-registration of angiography and intravascular ultrasound images. In: Fichtinger, G., Martel, A., Peters, T. (eds.) Medical Image Computing and Computer-Assisted Intervention - MICCAI 2011. pp. 161-168. Springer Berlin Heidelberg, Berlin, Heidelberg (2011)

12. Wang, P., Ecabert, O., Chen, T., Wels, M., Rieber, J., Ostermeier, M., Comaniciu, D.: Image-based co-registration of angiography and intravascular ultrasound images. In: IEEE Transactions on Medical Imaging. vol. 32, pp. 2238-2249 (2013)

13. Yan, B., Jiang, Y., Sun, P., Wang, D., Yuan, Z., Luo, P., Lu, H.: Towards grand unification of object tracking. In: Avidan, S., Brostow, G., Ciss'e, M., Farinella, G.M., Hassner, T. (eds.) Computer Vision - ECCV 2022. pp. 733-751. Springer

Nature Switzerland, Cham (2022) 14. Yan, B., Peng, H., Fu, J., Wang, D., Lu, H.: Learning spatio-temporal transformer for visual tracking. In: 2021 IEEE/CVF International Conference on Computer Vision (ICCV). pp. 10428-10437 (2021)

[21] Chon, Min Ku, Kook Jin Chun, Dae Sung Lee, Soo Yong Lee, Jongmin Hwang, Sang Hyun Lee, Ki Won Hwang, Jeong Su Kim, Young Huyn Park, and June Hong Kim. "Radiation Reduction during Percutaneous Coronary Intervention." Medicine 96, no. 30 (July 28, 2017): e7517. https://doi.org/10.1097/MD.0000000000007517.

[22] Yan, Bin, Houwen Peng, Jianlong Fu, Dong Wang, and Huchuan Lu. "Learning Spatio-Temporal Transformer for Visual Tracking." arXiv, March 31, 2021. https://doi.org/10.48550/arXiv.2103.17154.

[23] He, Kaiming, Xiangyu Zhang, Shaoqing Ren, and Jian Sun. "Deep Residual Learning for Image Recognition." arXiv, December 10, 2015. https://doi.org/10.48550/arXiv.1512.03385.

[24] Teixeira, Brian, Birgi Tamersoy, Vivek Singh, and Ankur Kapoor. "Adaloss: Adaptive Loss Function for Landmark Localization." arXiv, August 2, 2019. https://doi.org/10.48550/arXiv.1908.01070.

[25] Teed, Zachary, and Jia Deng. "RAFT: Recurrent All-Pairs Field Transforms for Optical Flow." arXiv, August 25, 2020. https://doi.org/10.48550/arXiv.2003.12039.

[26] Yan, Bin, Yi Jiang, Peize Sun, Dong Wang, Zehuan Yuan, Ping Luo, and Huchuan Lu. "Towards Grand Unification of Object Tracking." arXiv, July 28, 2022. https://doi.org/10.48550/arXiv.2207.07078.

[27] Wang, Qiang, et al. "Fast online object tracking and segmentation: A unifying approach." Proceedings of the IEEE/CVF conference on Computer Vision and Pattern Recognition. 2019

[28] Zhang, Guifang, et al. "A Temporary Transformer Network for Guide- Wire Segmentation", In: 14th International Congress on Image and Signal Processing, BioMedical Engineering and Informatics (CISP-BMEI), October 23, 2021. https://doi.org/10.1109/CISP-BMEI53629.2021.9624350.

**Claims**

1.  A computer-implemented method of tracking a target in medical imaging data, the method comprising:

    - determining (405) an encoded representation (112) of a search image (102) of the medical imaging data in a feature space using a feature encoding network (151), the search image (102) depicting a target and a surrounding of the target,
    - determining (410) encoded representations (111) of one or more template images (101) of the medical imaging data in the feature space using the feature encoding network (151), the one or more template images (101) depicting the target,
    - determining (415) fused features by fusing the encoded representations of the one or more template images and the encoded representation of the search image using a fusion network (153),
    - based on the fused features, determining (420) a position prediction (131) of the target in the search image (102),
    - based on the fused features, determining (425) a segmentation (132) of a context of the target in the search image (102), **characterized by**
    - refining (430) the position prediction of the target based on the segmentation (132) of the context of the target.

2. The method of claim 1, further comprising:

   - determining (426) an optical flow (162) based on the segmentation (132) of the context and one or more previous segmentations (135) of the context,

   wherein the position prediction (131) of the target is refined (430) based on the optical flow (162).

3. The method of claim 2, wherein said refining (of the position prediction comprises:

   - applying a refinement network (156) to the optical flow (162) and the position prediction (131) of the target.

4. The method of claims 2 or 3, further comprising:

   - refining (435) the segmentation of the context of the target based on the optical flow.

5. The method of claim 4,
   wherein the segmentation of the context of the target is refined further based on a vessel segmentation.

6. The method of claim 4 or 5,
   wherein the segmentation of the context of the target is refined in a spatial-temporal mask refinement block, and the spatial-temporal mask refinement block combines both the segmentation (132) of the context and the optical flow (162).

7. The method of any one of the preceding claims,

   wherein the target is a tip of an interventional medical instrument,
   wherein the context is a body of the interventional medical instrument extending away from the tip.

8. The method of any one of claims 1 to 7,
   wherein the context are predefined anatomical features in a surrounding of the target.

9. The method of any one of the preceding claims,
   wherein each of the one or more template images has at least one of a lower resolution or a smaller size than the search image.

10. The method of any one of the preceding claims,
    wherein the fused features are determined using a vision transformer network (153).

11. A processing device (301) comprising a processor (302) and a memory (303) storing program code, the processor being configured to load and execute the program code, the processor, upon executing the program code, being configured to perform the method of any one of claims 1 to 10.

12. A program code executable by a processor, the processor, upon executing the program code, being configured to perform the method of any one of claims 1 to 10.


**Patentansprüche**

1. Computerimplementiertes Verfahren zum Verfolgen eines Ziels in medizinischen Bildgebungsdaten, wobei das Verfahren umfasst:

   - Bestimmen (405) einer codierten Repräsentation (112) eines Suchbildes (102) der medizinischen Bildgebungsdaten in einem Merkmalsraum unter Verwendung eines Merkmalcodiernetzwerks (151), wobei das Suchbild (102) ein Ziel und eine Umgebung des Ziels darstellt,
   - Bestimmen (410) codierter Repräsentationen (111) eines oder mehrerer Vorlagenbilder (101) der medizinischen Bildgebungsdaten in dem Merkmalsraum unter Verwendung des Merkmalscodiernetzwerks (151), wobei das eine oder die mehreren Vorlagenbilder (101) das Ziel darstellen,
   - Bestimmen (415) fusionierter Merkmale durch Fusionieren der codierten Repräsentationen des einen oder der mehreren Vorlagenbilder und der codierten Repräsentation des Suchbildes unter Verwendung eines Fusionsnetzwerks (153),

- Bestimmen (420) einer Positionsvorhersage (131) des Ziels in dem Suchbild (102) basierend auf den fusionierten Merkmalen,

- Bestimmen (425) einer Segmentierung (132) eines Kontexts des Ziels in dem Suchbild (102) basierend auf den fusionierten Merkmalen, **gekennzeichnet durch**

- Verfeinern (430) der Positionsvorhersage des Ziels basierend auf der Segmentierung (132) des Kontexts des Ziels.

2. Verfahren nach Anspruch 1, ferner umfassend:

   - Bestimmen (426) eines optischen Flusses (162) basierend auf der Segmentierung (132) des Kontexts und einer oder mehreren vorherigen Segmentierungen (135) des Kontexts,

   wobei die Positionsvorhersage (131) des Ziels basierend auf dem optischen Fluss (162) verfeinert (430) wird.

3. Verfahren nach Anspruch 2, wobei das Verfeinern der Positionsvorhersage umfasst:

   - Anwenden eines Verfeinerungsnetzwerks (156) auf den optischen Fluss (162) und die Positionsvorhersage (131) des Ziels.

4. Verfahren nach Anspruch 2 oder 3, ferner umfassend:

   - Verfeinern (435) der Segmentierung des Kontexts des Ziels basierend auf dem optischen Fluss.

5. Verfahren nach Anspruch 4,
   wobei die Segmentierung des Kontexts des Ziels ferner basierend auf einer Gefäßsegmentierung verfeinert wird.

6. Verfahren nach Anspruch 4 oder 5,
   wobei die Segmentierung des Kontexts des Ziels in einem Raum-Zeit-Masken-Verfeinerungsblock verfeinert wird und der Raum-Zeit-Masken-Verfeinerungsblock sowohl die Segmentierung (132) des Kontexts als auch den optischen Fluss (162) kombiniert.

7. Verfahren nach einem der vorhergehenden Ansprüche,

   wobei das Ziel eine Spitze eines interventionellen medizinischen Instruments ist,
   wobei der Kontext ein Körper des interventionellen medizinischen Instruments ist, der sich von der Spitze weg erstreckt.

8. Verfahren nach einem der Ansprüche 1 bis 7,
   wobei der Kontext vordefinierte anatomische Merkmale in einer Umgebung des Ziels ist.

9. Verfahren nach einem der vorhergehenden Ansprüche,
   wobei jedes des einen oder der mehreren Vorlagenbilder mindestens eines von einer niedrigeren Auflösung oder einer kleineren Größe als das Suchbild aufweist.

10. Verfahren nach einem der vorhergehenden Ansprüche,
    wobei die fusionierten Merkmale unter Verwendung eines Sichttransformatornetzwerks (153) bestimmt werden.

11. Verarbeitungsvorrichtung (301), umfassend einen Prozessor (302) und einen Speicher (303), der Programmcode speichert, wobei der Prozessor dazu ausgelegt ist, den Programmcode zu laden und auszuführen, wobei der Prozessor dazu ausgelegt ist, bei Ausführung des Programmcodes das Verfahren nach einem der Ansprüche 1 bis 10 durchzuführen.

12. Programmcode, der durch einen Prozessor ausführbar ist, wobei der Prozessor dazu ausgelegt ist, bei Ausführung des Programmcodes das Verfahren nach einem der Ansprüche 1 bis 10 durchzuführen.

**EP 4 425 428 B1**

**Revendications**

1. Procédé mis en œuvre par ordinateur permettant de suivre une cible dans des données d'imagerie médicale, le procédé comprenant :

   - la détermination (405) d'une représentation codée (112) d'une image de recherche (102) des données d'imagerie médicale dans un espace de caractéristiques au moyen d'un réseau de codage de caractéristiques (151), l'image de recherche (102) représentant une cible et un environnement de la cible,
   - la détermination (410) de représentations codées (111) d'une ou de plusieurs images modèles (101) des données d'imagerie médicale dans l'espace de caractéristiques à l'aide du réseau de codage de caractéristiques (151), les une ou plusieurs images modèles (101) représentant la cible,
   - la détermination (415) de caractéristiques fusionnées en fusionnant les représentations codées des une ou plusieurs images modèles et la représentation codée de l'image de recherche à l'aide d'un réseau de fusion (153),
   - sur la base des caractéristiques fusionnées, la détermination (420) d'une prédiction de position (131) de la cible dans l'image de recherche (102),
   - sur la base des caractéristiques fusionnées, la détermination (425) d'une segmentation (132) d'un contexte de la cible dans l'image de recherche (102), **caractérisée par**
   - l'affinement (430) de la prédiction de position de la cible sur la base de la segmentation (132) du contexte de la cible.

2. Procédé selon la revendication 1, comprenant en outre :

   - la détermination (426) d'un flux optique (162) basé sur la segmentation (132) du contexte et une ou plusieurs segmentations précédentes (135) du contexte,

   dans lequel la prédiction de position (131) de la cible est affinée (430) sur la base du flux optique (162).

3. Procédé selon la revendication 2, dans lequel ledit affinement de la prédiction de position comprend :

   - l'application d'un réseau d'affinement (156) au flux optique (162) et à la prédiction de position (131) de la cible.

4. Procédé selon la revendication 2 ou 3, comprenant en outre :

   - l'affinement (435) de la segmentation du contexte de la cible sur la base du flux optique.

5. Procédé selon la revendication 4,
   dans lequel la segmentation du contexte de la cible est encore affinée sur la base d'une segmentation de vaisseau.

6. Procédé selon la revendication 4 ou 5,
   dans lequel la segmentation du contexte de la cible est affinée dans un bloc d'affinement de masque spatio-temporel, et le bloc d'affinement de masque spatio-temporel combine à la fois la segmentation (132) du contexte et le flux optique (162).

7. Procédé selon l'une quelconque des revendications précédentes,

   dans lequel la cible est une pointe d'un instrument médical interventionnel,
   dans lequel le contexte est un corps de l'instrument médical interventionnel s'étendant à l'opposé de la pointe.

8. Procédé selon l'une quelconque des revendications 1 à 7,
   dans lequel le contexte est des caractéristiques anatomiques prédéfinies dans un environnement de la cible.

9. Procédé selon l'une quelconque des revendications précédentes,
   dans lequel chacune des une ou plusieurs images modèles présente une résolution inférieure et/ou une taille inférieure à celle de l'image de recherche.

10. Procédé selon l'une quelconque des revendications précédentes,
    dans lequel les caractéristiques fusionnées sont déterminées à l'aide d'un réseau de transformateurs de vision (153).

**12**

**11.** Dispositif de traitement (301) comprenant un processeur (302) et une mémoire (303) stockant un code de programme, le processeur étant configuré pour charger et exécuter le code de programme, le processeur, lors de l'exécution du code de programme, étant configuré pour réaliser le procédé selon l'une quelconque des revendications 1 à 10.

**12.** Code de programme exécutable par un processeur, le processeur, lors de l'exécution du code de programme, étant configuré pour réaliser le procédé selon l'une quelconque des revendications 1 à 10.

FIG 1A

FIG 1B

# FIG 2

Template 101 · 151 · 111 · 153 · 154 Tip-Decoder · 131 · 170 · 156 Tip Refinement · 171 tip location on frame t+1

Res-50 · flatten · 64 x 64

Search: Frame t+1 · 151 · 112 · Transformer Feature Fusion · 155 Body-Decoder · 132

Res-50 · flatten · 160 x 160 · 102

- ⊏⃞⊐ Trainable networks
- ☐ Features
- ☐ Network with weight frozen
- ⊕ concatenation

Context guided flow predictor · 161

135 · Catheter Mask Prediction Frame t

Predicted Flow · 162 · 163

Spatial-Temporal Mask refinement · 164

Vessel Segmentation

Catheter Mask Prediction Frame t+1 · 172

191 · 192

100

EP 4 425 428 B1

15

## FIG 3

## FIG 4

FIG 5

```
┌─────────────────┐
│                 │─ 405
└─────────────────┘
         │
         ▼
┌─────────────────┐
│                 │─ 410
└─────────────────┘
         │
         ▼
┌─────────────────┐
│                 │─ 415
└─────────────────┘
         │
         ▼
┌─────────────────┐
│                 │─ 420
└─────────────────┘
         │
         ▼
┌─────────────────┐
│                 │─ 425
└─────────────────┘
         │
         ▼
┌─────────────────┐
│                 │─ 426
└─────────────────┘
         │
         ▼
┌─────────────────┐
│                 │─ 430
└─────────────────┘
         │
         ▼
┌─────────────────┐
│                 │─ 435
└─────────────────┘
```

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ARAKI, T.** ; **IKEDA, N** ; **DEY, N** ; **CHAKRABORTY, S** ; **SABA, L** ; **KUMAR, D** ; **GODIA, E.C.** ; **JIANG, X** ; **GUPTA, A** ; **RADEVA, P**. *A comparative approach of four different image registration techniques for quantitative assessment of coronary artery calcium lesions using intravascular ultrasound.*, 2015, vol. 118, 158-172
- Signature verification using a "siamese" time delay neural network.. **BROMLEY, J.** ; **GUYON, I** ; **LECUN, Y** ; **ŠACKINGER, E** ; **SHAH, R**. Advances in Neural Information Processing Systems.. 1993, vol. 6
- **CHEN, Q** ; **WU, Q** ; **WANG, J.** ; **HU, Q** ; **HU, T** ; **DING, E** ; **CHENG, J.** ; **WANG, J.** Mixformer: Mixing features across windows and dimensions.. *Proceedings of the IEEE/CVF Conference on Computer Vision and Pattern Recognition (CVPR).*, 2022, 5249-5259
- Robust landmarkbased stent tracking in x-ray fluoroscopy.. **HUANG, L.** ; **LIU, Y** ; **CHEN, L** ; **CHEN, E.Z.** ; **CHEN, X** ; **SUN, S**. Computer Vision - ECCV 2022.. Springer Nature, 2022, 201-216
- **LI, B.** ; **YAN, J.** ; **WU, W** ; **ZHU, Z** ; **HU, X**. High performance visual tracking with siamese region proposal network.. *2018 IEEE/CVF Conference on Computer Vision and Pattern Recognition*, 2018, 8971-8980
- Cycle ynet: Semisupervised tracking of 3d anatomical landmarks. **LIN, J.** ; **ZHANG, Y** ; **AMADOU, A.A** ; **VOIGT, I** ; **MANSI, T.** ; **LIAO, R.** Machine Learning in Medical Imaging.. Springer International Publishing, 2020, 593-602
- **MA, H** ; **SMAL, I.** ; **DAEMEN, J.** ; **VAN WALSUM, T**. *Dynamic coronary roadmapping via catheter tip tracking in x-ray fluoroscopy with deep learning based bayesian filtering*, 2020, vol. 61, 101634
- **PIAYDA, K.** ; **KLEINEBRECHT, L.** ; **AFZAL, S** ; **BULLENS, R** ; **TER HORST, I** ; **POLZIN, A** ; **VEULEMANS, V.** ; **DANNENBERG, L.** ; **WIMMER, A.C** ; **JUNG, C**. *Dynamic coronary roadmapping during percutaneous coronary intervention: a feasibility study*, 2018, vol. 23, 36
- Raft: Recurrent all-pairs field transforms for optical flow.. **TEED, Z.** ; **DENG, J.** Computer Vision - ECCV 2020. Springer International Publishing, 2020, 402-419
- Attention is all you need.. **VASWANI, A.** ; **SHAZEER, N** ; **PARMAR, N** ; **USZKOREIT, J.** ; **JONES, L.** ; **GOMEZ, A.N.** ; **KAISER, L.U.** ; **POLOSUKHIN, I.** Advances in Neural Information Processing Systems.. Curran Associates, Inc., 2017, vol. 30
- Image-based device tracking for the co-registration of angiography and intravascular ultrasound images.. **WANG, P** ; **CHEN, T** ; **ECABERT, O** ; **PRUMMER, S** ; **OSTERMEIER, M** ; **COMANICIU, D.** Medical Image Computing and Computer-Assisted Intervention - MICCAI 2011. Springer, 2011, 161-168
- **WANG, P** ; **ECABERT, O** ; **CHEN, T** ; **WELS, M.** ; **RIEBER, J.** ; **OSTERMEIER, M** ; **COMANICIU, D**. Image-based co-registration of angiography and intravascular ultrasound images. *IEEE Transactions on Medical Imaging*, 2013, vol. 32, 2238-2249
- Towards grand unification of object tracking.. **YAN, B.** ; **JIANG, Y** ; **SUN, P.** ; **WANG, D** ; **YUAN, Z.** ; **LUO, P** ; **LU, H.** Computer Vision - ECCV. Springer, 2022, 733-751
- **YAN, B.** ; **PENG, H** ; **FU, J.** ; **WANG, D** ; **LU, H**. Learning spatio-temporal transformer for visual tracking. *2021 IEEE/CVF International Conference on Computer Vision (ICCV).*, 2021, 10428-10437
- **CHON, MIN KU** ; **KOOK JIN CHUN** ; **DAE SUNG LEE** ; **SOO YONG LEE** ; **JONGMIN HWANG** ; **SANG HYUN LEE** ; **KI WON HWANG** ; **JEONG SU KIM** ; **YOUNG HUYN PARK** ; **JUNE HONG KIM**. Radiation Reduction during Percutaneous Coronary Intervention. *Medicine*, 28 July 2017, vol. 96 (30), e7517, https://doi.org/10.1097/MD.0000000000007517
- **YAN, BIN** ; **HOUWEN PENG** ; **JIANLONG FU** ; **DONG WANG** ; **HUCHUAN LU**. Learning Spatio-Temporal Transformer for Visual Tracking. *arXiv*, 31 March 2021, https://doi.org/10.48550/arXiv.2103.17154
- **HE, KAIMING** ; **XIANGYU ZHANG** ; **SHAOQING REN** ; **JIAN SUN**. Deep Residual Learning for Image Recognition.. *arXiv*, 10 December 2015, https://doi.org/10.48550/arXiv.1512.03385
- **TEIXEIRA, BRIAN** ; **BIRGI TAMERSOY** ; **VIVEK SINGH** ; **ANKUR KAPOOR.** Adaloss: Adaptive Loss Function for Landmark Localization. *arXiv*, 02 August 2019, https://doi.org/10.48550/arXiv.1908.01070

- **TEED, ZACHARY** ; **JIA DENG**. RAFT: Recurrent All-Pairs Field Transforms for Optical Flow. *arXiv*, 25 August 2020, https://doi.org/10.48550/ar-Xiv.2003.12039
- **YAN, BIN** ; **YI JIANG** ; **PEIZE SUN** ; **DONG WANG** ; **ZEHUAN YUAN** ; **PING LUO** ; **HUCHUAN LU**. Towards Grand Unification of Object Tracking. *arXiv*, 28 July 2022, https://doi.org/10.48550/ar-Xiv.2207.07078
- **WANG, QIANG et al.** Fast online object tracking and segmentation: A unifying approach. *Proceedings of the IEEE/CVF conference on Computer Vision and Pattern Recognition*, 2019
- **ZHANG, GUIFANG et al.** A Temporary Transformer Network for Guide- Wire Segmentation. *International Congress on Image and Signal Processing, BioMedical Engineering and Informatics (CISP-BMEI)*, 23 October 2021, https://doi.org/10.1109/CISP-BMEI53629.2021.9624350